# EUROPÄISCHE PATENTANMELDUNG

(11) **EP 3 406 218 A1**
(43) Veröffentlichungstag der Anmeldung: **28.11.2018**
(21) Anmeldenummer: 17172876.9
(22) Anmeldetag: 24.05.2017
(51) Int. Cl.: A61B 18/14, A61B 18/00

(54) **KOAGULATIONS- UND DISSEKTIONS-INSTRUMENT MIT STIFTELEKTRODEN**

(71) Anmelder: Erbe Elektromedizin GmbH, 72072 Tübingen (DE)
(72) Erfinder: Nold, Bernhard Tobias, 72070 Tübingen (DE); Weiler, Rolf, 72074 Tuebingen (DE); Selig, Peter, 72147 Nehren (DE); Fischer, Klaus, 72202 Nagold (DE); Blobel, Lars, 72119 Ammerbuch (DE)
(74) Vertreter: Rüger Abel

(57) **Zusammenfassung**

Das erfindungsgemäße Instrument (10) weist eine Schenkelanordnung (13) mit zwei Schenkeln (22, 23) auf, die an ihren der Gegenschenkelanordnung (14) zugewandten Seite gerundete oder auch flache Elektrodenflächen (22a, 23a) aufweisen. Die Gegenschenkelanordnung (14) umfasst wenigstens eine Gegenelektrodenfläche (25a), die ebenfalls gerundet oder flach ausgebildet ist. Eine der Elektrodenflächen (22a, 23a) weist eine vorzugsweise einseitig von einem Isolator (27) eingefasste Randkante (28) auf, die der benachbarten Elektrodenfläche (22a) der Schenkelanordnung (13) benachbart ist. Die Gegenelektrodenfläche (25a) weist gleichfalls eine einem Isolator (30) benachbarte Randkante (31) auf, die der Randkante (28) zugewandt ist. Die Randkanten (28, 31) der als Koagulationsflächen dienenden Elektrodenflächen (23a, 25a) sind einander so nahe (0,1 mm bis 0,75 mm), dass sie Schneidkanten bilden, die im Idealfalle schon mit sonst nur zur Koagulation tauglichen niedrigen Spannungen eine Schneidwirkung entfalten. Mit diesem Konzept lassen sich äußerst filigrane Fusionsinstrumente mit geringer thermischen Trägheit und sehr guter Fusions- und Schneidwirkung bereitstellen.

## Beschreibung

Die Erfindung betrifft ein elektrochirurgisches Instrument, insbesondere zur Gefäßfusion.

Elektrochirurgische Instrumente dienen in der Praxis zum Trennen von biologischem Gewebe und zur blutstillenden Versorgung der Schnittsäume. Insbesondere können elektrochirurgische Instrumente zum Verschließen von Gefäßen, beispielsweise Blutgefäßen oder anderen Hohlorganen eingesetzt werden. Das Verschließen der Gefäße erfolgt typischerweise, indem das zu fusionierende Gefäß zwischen Schenkeln eines zangenartigen Instruments eingeklemmt und dann bestromt wird, so dass ein Koagulationseffekt eintritt. Außerdem kann das verschlossene Gefäß an der Verschlussstelle z.B. durch ein mechanisches oder elektrisches Messer durchtrennt werden.

Aus der US 5,944,718 ist ein Fusionsinstrument bekannt, das zwei stiftartige, in fester räumlicher Beziehungen parallel zueinander gehaltene Elektroden mit Rundquerschnitt aufweist. Den beiden Elektroden ist eine Gegenelektrode zugeordnet, die den Abstand zwischen den beiden stiftartigen Elektroden überbrückt und zur Gefäßfusion zwei konvex gerundete Gegenelektrodenabschnitte aufweist, zwischen denen ein flacherer Elektrodenabschnitt angeordnet ist. Dieses Instrument kann in dieser Konfiguration zur Fusion von Gefäßen genutzt werden.

Die Gegenelektrode dieses Instruments ist an einem Halter so drehbar befestigt, dass die konvex gerundeten Elektrodenflächen der Gegenelektrode von den Elektroden weg gedreht werden können. Stattdessen ist dann eine schmale Kante aktiv, die zwischen die Rundstabelektroden eintauchen kann. An die schmale Kante schließen sich konkave Flankenbereiche an.

Weiter ist aus der EP 1 089 664 B1 ein Instrument zur Gefäßfusion und -Durchtrennung bekannt, das Branchen aus U-förmig gebogenen Runddrähten oder gezahnten Drähten aufweist. Elektrode und Gegenelektrode sind dabei fluchtend so zueinander angeordnet, dass ein zwischen der Elektrode und der Gegenelektrode gefasstes und koaguliertes Gefäß durch eine axial vorschiebbare ebene Schneidelektrode aufgetrennt werden kann.

Außerdem ist aus der EP 1 051 120 B1 ein Fusionsinstrument mit bügelförmiger Drahtelektrode und ebenso bügelförmiger, dazu fluchtender Gegenelektrode bekannt. Zwischen den beiden Schenkeln der bügelförmigen Elektrode und Gegenelektrode ist ein breiter Freiraum vorhanden. Durch diesen Freiraum hindurch kann eine Draht-Schneidelektrode tauchen, um einen Schnitt durch ein fusioniertes Gefäß zu bewirken.

Aus der US 5,269,780 ist ein Instrument bekannt, das zum Schneiden oder Koagulieren von biologischem Gewebe dient. Es weist zwei im Querschnitt runde Elektroden auf, die im Abstand parallel zueinander gehalten sind. Als Gegenelektrode ist eine Schneidelektrode mit Rundquerschnitt, jedoch geringerem Durchmesser als die beiden Elektroden, vorgesehen. Die Schneidelektrode kann zwischen den beiden Elektroden durch tauchen, weswegen sich dieses Instrument zum Trennen von Gewebe, nicht aber zur Gefäßfusion eignet.

Die WO 2016/088017 A1 beschreibt ein Koagulationsinstrument mit zueinander fluchtenden Branchen, die einen Rundquerschnitt aufweisen. Die Elektroden sind mit Isolator-Inlays versehen, die und zueinander asymmetrisch angeordnet sind. Damit sollen lokale Stromkonzentrationen zur Herbeiführung eines Schneideffekts erzeugt werden.

Weiter offenbart die US 6,152,923 ein Fusionsinstrument mit sechs paarweise fluchtend angeordneten Flachelektroden. Während das mittlere Elektrodenpaar zur Gefäßdurchtrennung dient, bilden die zu beiden Seiten angeordneten Elektrodenpaare Fusionselektroden, die zum Verschluss der voneinander getrennten Enden des geschnittenen Gefäßes dienen.

Bei der Entwicklung von Gefäßfusionsinstrumenten kommt es darauf an, eine möglichst schnelle und sichere Versiegelung von Gefäßen zu erreichen. Zugleich gibt es einen Trend zur Miniaturisierung bis hin zum laparoskopischen Einsatz von Fusionsinstrumenten oder zur Nutzung in einem Trokar oder einem Katheter. Es hat sich aber gezeigt, dass sich bekannte Fusionsinstrumente bei einer gewünschten Verkleinerung der Abmessungen nicht einfach maßstäblich verkleinern lassen, denn auch verkleinerte Instrumente müssen in der Lage sein, gleich große Gefäße zu behandeln wie bisher. Besonderes Augenmerk liegt dabei auf der Sicherheit des Gefäßverschlusses. Soll ein elektrischer Schnitt durchgeführt werden, können infolge der Höhe der üblicherweise erforderlichen Schneidspannung bei miniaturisierten Instrumenten Isolationsprobleme auftreten. Außerdem kann die Miniaturisierung zu einer erheblichen Flexibilität, d.h. mechanischen Nachgiebigkeit der Branchen führen. Dies ist aber wegen der angestrebten Miniaturisierung und der damit einhergehenden erhöhten Genauigkeitsanforderung problematisch.

Darauf aufbauend ist es Aufgabe der Erfindung, ein Konzept für ein Fusionsinstrument anzugeben, mit dem sich besonders kleine Bauformen und gegebenenfalls auch weitere Vorteile realisieren lassen.

Diese Aufgabe wird mit dem Instrument nach Anspruch 1 gelöst:

Bei dem erfindungsgemäßen Instrument handelt es sich um ein Koagulations- und Dissektions-Instrument mit Stiftelektroden, dessen Schenkel durch eine Schenkelanordnung und eine Gegenschenkelanordnung gebildet sind. Die Schenkelanordnung weist einen ersten Schenkel mit einer ersten Elektrodenfläche und einen zweiten Schenkel mit einer zweiten Elektrodenfläche auf, die in fester räumlicher Beziehung zueinander gehalten sind. Die Gegenschenkelanordnung weist wenigstens einen Gegenschenkel auf. Wenigstens einer von dem ersten Schenkel, dem zweiten Schenkel und dem Gegenschenkel weist eine Elektrodenfläche auf, die zu einer Mittellinie E außermittig angeordnet ist, die durch Bewegungsrichtung R des Schenkels oder Gegenschenkels und dessen Querschnittsmittelpunkt festgelegt ist. Dadurch kann dem Gegenschenkel eine Doppelfunktion zugeordnet werden: Mit dem ersten Schenkel bildet er einen Fusionsspalt aus und mit dem zweiten Schenkel bildet er einen Schneidspalt. Weil aber dem wenigstens einen vorzugsweise stiftförmigen Gegenschenkel wenigstens zwei ebenfalls vorzugsweise stiftförmige Schenkel gegenüberstehen, können sich Schenkel und Gegenschenkel in Bezug aufeinander ausrichten, wobei sich der Gegenschenkel zwischen den Schenkel zentriert. Damit können auch bei einer gewissen federnden Nachgiebigkeit der Schenkel und des wenigstens einen Gegenschenkels präzise Arbeitsergebnisse erreicht werden, denn die Schenkel und Gegenschenkel stützen sich beim Schließen aneinander ab und geben sich gegenseitig seitliche Führung.

Die Schenkelanordnung und die Gegenschenkelanordnung sind so ausgebildet und relativ zueinander so positioniert und bewegbar, dass sie biologisches Gewebe, insbesondere ein Gefäß, zwischen einander fassen und zusammendrücken, insbesondere so zusammendrücken können, dass das im Gefäß vorhandene Lumen vollständig geschlossen wird. Vorzugsweise sind die Schenkelanordnung und die Gegenschenkelanordnung dabei so ausgebildet und gehalten, dass sie beim Schließen der Branchen, d.h. wenn sie aufeinander zu bewegt werden, gegeneinander versetzt aufeinander treffen. Vorzugsweise weist dazu die Gegenschenkelanordnung wenigstens einen Schenkel auf, der breiter als der Abstand zwischen dem ersten und dem zweiten Schenkel ausgebildet ist, so dass der Gegenschenkel nicht durch die zwischen den Schenkeln gebildete Lücke passt. Sind mehrere Gegenschenkel vorhanden, ist der Abstand zwischen den Gegenschenkeln vorzugsweise kleiner als die Breite eines Schenkels. Die Breiten der oben genannten Abstände werden dabei jeweils quer zur Bewegungsrichtung der Schenkel beim Öffnen und Schließen des Instruments gemessen.

Vorzugsweise weist der zweite Schenkel der Schenkelanordnung einen elektrisch leitenden Elektrodenabschnitt und einen isolierten Abschnitt auf, der auf der dem ersten Schenkel zugewandten Seite des zweiten Schenkels angeordnet ist. Außerdem weist der Gegenschenkel vorzugsweise einen elektrisch leitenden Gegenelektrodenabschnitt und einen elektrisch isolierten Gegenabschnitt auf, der auf der dem ersten Schenkel abgewandten Seite angeordnet ist. Der elektrisch leitende Elektrodenabschnitt des zweiten Schenkels der Schenkelanordnung kann in Nachbarschaft zu dem isolierten Abschnitt eine Kante aufweisen. Dadurch kann an den Rändern der Elektroden eine Stromkonzentration herbeigeführt werden, durch die ein Gewebeschnitt herbeigeführt werden kann.

Bei dem erfindungsgemäßen Instrument weist der Gegenschenkel z.B. einen Kreisquerschnitt auf, wobei der Abstand zwischen dem ersten Schenkel und dem zweiten Schenkel voneinander geringer ist, als der Durchmesser des Gegenschenkels. Es kann so sichergestellt werden, dass die beiden Schenkel und der zugeordnete Gegenschenkel übereinstimmende Steifigkeit bzw. Nachgiebigkeit haben. Dabei ist die Querschnittsfläche des Gegenschenkels vorzugsweise höchstens so groß, wie die Summe der beiden Querschnittsflächen der beiden Schenkel. Es ist dabei von Vorteil, wenn die Querschnittsfläche der Gegenelektrode höchstens so groß ist, wie die Summe der beiden Querschnittsflächen der beiden Elektroden. Insbesondere wird damit eine einseitige Kühlung des zu fusionierenden Gefäßes vermieden. Die Koagulation wirkt besonders gleichmäßig. Dabei ist es vorteilhaft, wenn die beiden Schenkel und der Gegenschenkel gleiches thermisches Verhalten zeigen, d.h. sich beim Einsatz mit gleicher Erwärmungs- und Abkühlrate erwärmen und abkühlen. Dazu ist es vorteilhaft, wenn die Schenkel und der Gegenschenkel eine vergleichbare Wärmekapazität aufweisen.

Der Kreisquerschnitt ist nur eine mögliche Ausführungsform. Die Schenkel und der Gegenschenkel können von der Kreisform abweichende Querschnitte aufweisen, wobei vorzugsweise im Kontaktbereich der Schenkel konvexe Oberflächen vorgesehen sind. Rückseitig, d.h. an der von dem Arbeitsspalt abliegenden Seite können die Schenkel durch einen oder mehrere Stege miteinander verbunden sein. Z.B. kann die Schenkelanordnung durch ein flaches, durchgangsloses Teil, z.B. aus Kunststoff, gebildet sein, das zwei im Abstand zueinander angeordnet streifenförmige, vorzugsweise gerundete Erhebungen aufweist.

Der Mittelpunkt des Gegenschenkels ist vorzugsweise auf einer Mittellinie angeordnet, zu denen auch die Schenkel der Schenkelanordnung auf verschiedenen Seiten in gleichen Abständen angeordnet sind. Die Schenkel können dabei auf gleicher Höhe, d.h. an der Mittellinie gespiegelt, oder auf unterschiedlicher Höhe angeordnet sein.

Bei einem bevorzugten Instrument können die erste Elektrodenfläche und die zweite Elektrodenfläche elektrisch untereinander verbunden sein. Dies trifft insbesondere zu, wenn die Schneidspannung mit der Koagulationsspannung übereinstimmt. Die physiologische Wirkung (Schneiden/Koagulieren) wird durch die Elektrodenform bestimmt. Das erfindungsgemäße Konzept ermöglicht die Anwendung niedriger Schneidspannungen von z.B. unter 300 Vₚ, vorzugsweise unter 250 Vₚ. Dies mildert sonst übliche Isolationsprobleme, die insbesondere bei der Miniaturisierung der Instrumente auftreten könnten. Das erfindungsgemäße Instrument kann für die laparoskopische Chirurgie hergerichtet werden, bei der nur wenig Bauraum, z.B. ein Durchmesser von lediglich 5mm für das Instrument zur Verfügung steht.

Bei einer bevorzugten Ausführungsform gehören zu der Gegenschenkelanordnung zwei Gegenschenkel, von denen einer nur zur Koagulation dient, wobei der andere zusätzlich eine Schneidfunktion erfüllt. Der Gegenschenkel erfüllt eine Schneid- und Koagulationsfunktion.

Vorzugsweise weist das Instrument zwei Schenkel und zwei Gegenschenkel auf, wobei die Schenkel der Schenkelanordnung und die Gegenschenkel der Gegenschenkelanordnung seitlich gegeneinander versetzt angeordnet sind. Schenkel und Gegenschenkel sind dadurch selbstzentrierend. Die Gegenelektroden legen dann mit den Elektroden zwei Koagulationsspalte und einen Schneidspalt fest, so dass an verschiedenen Positionen koaguliert und geschnitten wird.

Durch die genannte Maßnahme können die Elektroden und die Gegenelektrode(n) äußerst schlank und dabei hinsichtlich ihrer Fusionswirkung überraschend effizient ausgebildet werden. Vorzugsweise ist keine planare, an dem Arbeitsspalt mit dem Gewebe in Kontakt kommende Fläche vorgesehen. Insbesondere ist keine Paarung planarer Flächen vorgesehen, d.h. der Koagulationsspalt weist quer zu den Schenkeln ein nichtkonstantes Spaltmaß auf. Damit ist eine weitgehende Miniaturisierung möglich, wobei mit derart miniaturisierten Fusionsinstrumenten ebenso große Gefäße versiegelt werden können, wie zuvor mit größeren Instrumenten.

Die erfindungsgemäße Gestaltung der Schenkelanordnung und der Gegenschenkelanordnung stellt ein gegen Fertigungstoleranzen unempfindliches Konzept dar. Die Elektroden und Gegenelektroden sind einander beim Schließen gegenseitig zentrierend ausgebildet. Außerdem werden Gefäße durch die Elektroden und die wenigstens eine Gegenelektrode sehr fest gefasst, so dass die Gefahr des Verrutschens vermindert oder beseitigt ist.

Weiter ergibt das erfindungsgemäße Konzept eine besonders schmale Bauform, die insbesondere bei laparoskopischem oder endoskopischem Arbeiten dem Behandler eine gute Sicht auf das Koagulationsinstrument und das zu bearbeitende Gefäß ermöglicht.

Bei einer bevorzugten Ausführungsform weist die Gegenelektrode einen Kreisquerschnitt auf, wobei der Abstand zwischen der ersten Elektrode und der zweiten Elektrode voneinander geringer ist als der Durchmesser der Gegenelektrode. Damit wird eine sichere Versiegelung eines Gefäßes erzielt.

Alternativ kann die Gegenelektrode einen ein- oder mehreckigen Querschnitt aufweisen, wobei der Mittenabstand Am der beiden nichtkonkaven, vorzugsweise ebenen Abschnitte der Gegenelektrode vorzugsweise größer ist als der Abstand der beiden Elektroden voneinander. Auch damit wird ein schlankes sehr wirksames Fusionsinstrument erhalten.

Bei dem erfindungsgemäßen Instrument können die Schenkel der Schenkelanordnung und die Gegenschenkel der Gegenschenkelanordnung seitlich gegeneinander versetzt angeordnet sein. Dabei kann sich jeweils ein Schenkel eines Schenkelpaars auf einer mittig zwischen den Schenkeln des anderen Schenkelpaars liegenden Linie liegen. Beim Schließen des Instruments wird eine automatische Zentrierung der Schenkel zueinander erreicht. Damit ist ein sicheres Arbeiten auch mit Instrumenten möglich, die sehr kleine Abmessungen aufweisen und sehr filigran sind und/oder etwas federn.

Weitere Details vorteilhafter Ausführungsformen der Erfindung ergeben sich aus der Zeichnung, der Beschreibung oder Ansprüchen. Es zeigen:

Figur 1 das erfindungsgemäße Fusionsinstrument und sein speisendes Gerät, in einer schematisierten teilweise perspektivische Darstellung;

Figur 2 Elektroden und Gegenelektrode des Fusionsinstruments nach Figur 1, in schematischer Darstellung;

Figur 3 Elektroden und Gegenelektrode des Fusionsinstruments nach Figur 1 und 2, bei der Fusion eines Gefäßes in schematisierter Querschnittsdarstellung;

Figur 4 Elektroden und Gegenelektroden in einem vergrößerten Querschnitt zur Veranschaulichung von Maßbeziehungen;

Figur 5 bis 7 abgewandelte Schenkel- und Gegenschenkelanordnungen, jeweils in schematisierter Querschnittsdarstellung;

Figur 8 und 9 Prinzipschaltbilder zur Veranschaulichung der Spannungs- oder Strombeaufschlagung des Fusionsinstruments;

Figur 10 eine weiter abgewandelte Schenkel- und Gegenschenkelanordnung, jeweils in schematisierter Querschnittsdarstellung;

Figur 11 eine Schenkel- Gegenschenkelanordnung in schematisierter Querschnittsdarstellung mit einer Veranschaulichung der Bestromung in unterschiedlichen Betriebsphasen.

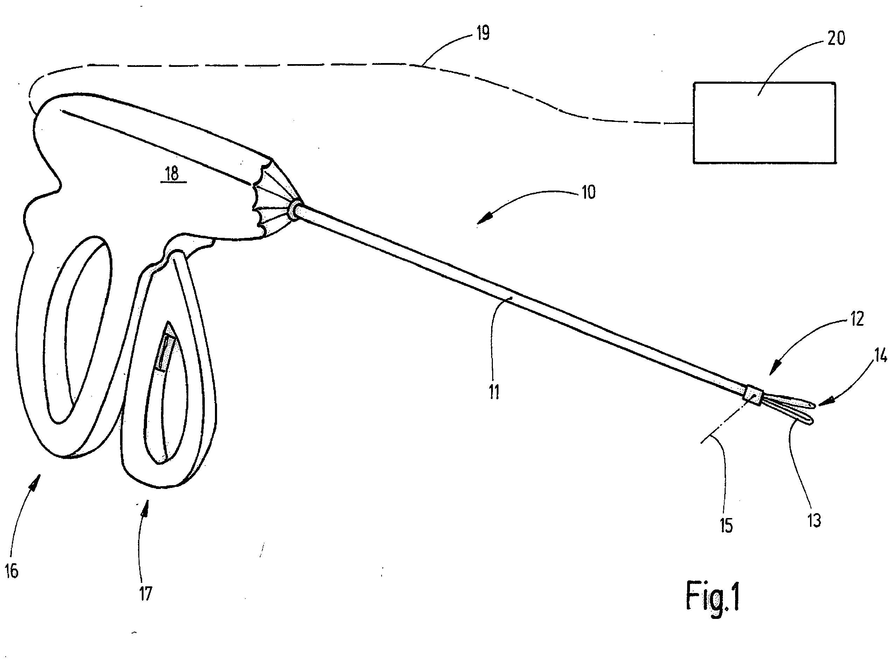

In Figur 1 ist ein Instrument 10 veranschaulicht, das zum Schließen und Versiegeln von Gefäßen eines menschlichen oder tierischen Patienten oder auch zum Trennen und/oder Koagulieren von Gewebe genutzt werden kann. Das Instrument kann für den offenchirurgischen Einsatz, als laparoskopisches Instrument oder als endoskopisches Instrument ausgebildet sein. Figur 1 veranschaulicht das Instrument 10 beispielhaft für den laparoskopischen Einsatz. Es weist dazu ein am distalen Ende eines Schafts 11 gehaltenes Werkzeug 12 auf, zu dem eine Schenkelanordnung 13 und eine Gegenschenkelanordnung 14 gehören. Die Schenkelanordnung 13 und/oder die Gegenschenkelanordnung 14 ist um eine quer zu dem Schaft 11 orientierte Achse 15 schwenkbar gehalten, so dass die Schenkelanordnung 13 und die Gegenschenkelanordnung 14 aufeinander zu und voneinander weg bewegt werden können. Zur gezielten Bewegung derselben ist ein Griff 16 vorgesehen, dem ein Betätigungshebel 17 zugeordnet ist. Durch gezielte Bewegung des Betätigungshebels 17 schließt das Werkzeug 12 und es können Gewebe, z.B. ein Gefäß, zwischen der Schenkelanordnung 13 und der Gegenschenkelanordnung 14 gehalten und geklemmt werden.

Während der Griff 16 Teile eines das proximale Ende des Schafts 11 aufnehmenden Gehäuses 18 ist oder sein kann, ist der Betätigungshebel 17 an diesem Gehäuse 18 schwenkbar gelagert oder anderweitig beweglich gehalten. Durch den Schaft 11 erstreckt sich wenigstens ein Betätigungselement zum Bewegen der Schenkelanordnung 13 und/oder der Gegenschenkelanordnung 14. Des Weiteren erstrecken sich elektrische Leitungen durch den Schaft 11, das Gehäuse 18 sowie durch ein Kabel 19, um das Werkzeug 12 mit einem Gerät 20 zum Betrieb des Instruments 10 zu verbinden.

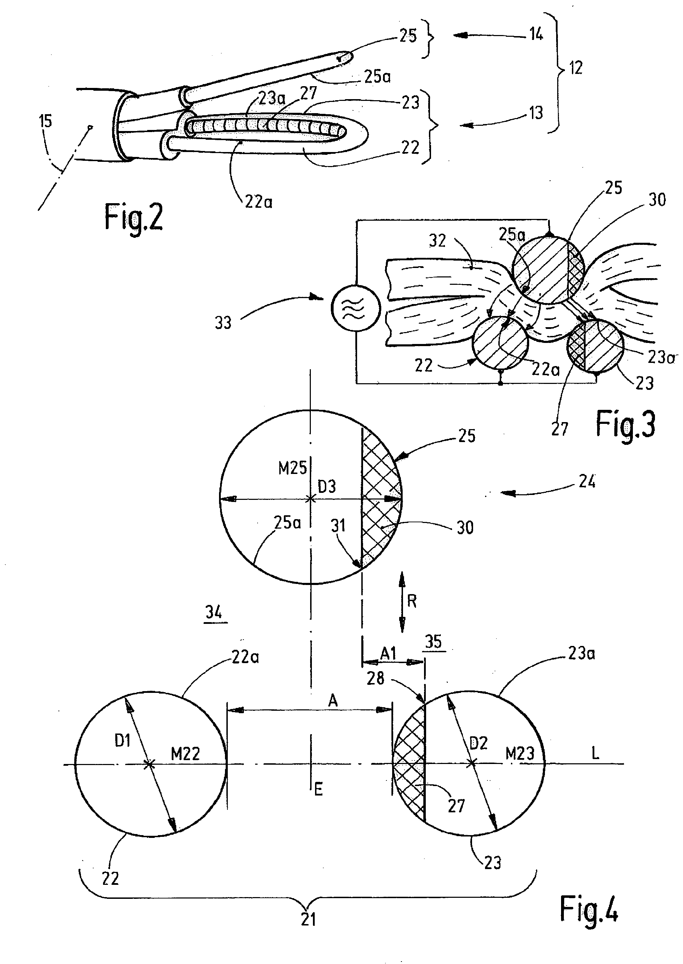

Das Werkzeug 12 ist in Figur 2 weiter veranschaulicht. Die untere Schenkelanordnung 13 weist zwei in Längsrichtung gerade oder leicht gekrümmte Schenkel 22, 23 auf, die z.B. durch Drähte oder Stäbe gebildet sind. Die Schenkel 22, 23 erstrecken sich vorzugsweise parallel zueinander in einem festen Abstand. Sie können einen konstanten, miteinander übereinstimmenden Querschnitt aufweisen und an ihrem distalen Ende untereinander durch einen z.B. u-förmigen Biegeabschnitt mechanisch und elektrisch oder durch einen elektrischen Isolator lediglich mechanisch miteinander verbunden sein. Im vorliegenden Ausführungsbeispiel ist die Schenkelanordnung 13 durch einen Drahtbügel gebildet. Die Schenkel 22, 23 können sich aber auch in distaler Richtung verjüngen, in einem spitzen Winkel zueinander angeordnet sein und/oder an ihrem distalen Ende voneinander getrennt sein. Unabhängig davon können die beiden Schenkel 22, 23 an ihrem distalen Ende alternativ auch unverbunden sein. Sie sind jedoch in fester räumlicher Beziehung, vorzugsweise parallel zueinander gehalten.

Die Schenkel 22, 23 weisen elektrisch leitfähige Elektrodenflächen 22a, 23a auf, die mit einem Pol einer elektrischen Quelle des Geräts 20 verbunden oder verbindbar sind, wie in Figur 3 veranschaulicht ist. Die Elektrodenflächen 22a, 23a werden bei diesem Ausführungsbeispiel von der nicht isolierten, frei liegenden Oberfläche der Schenkel 22, 23 gebildet. Nur einer der Schenkel 22, 23, hier der Schenkel 23, ist an seiner dem anderen Schenkel 22 zugewandten Seite mit einem Isolator 27 versehen, der sich in die Kontur des Schenkels 23 einfügt. Der Isolator 27 schließt direkt an eine Randkante 28 der Elektrodenfläche 23a an (Figur 4).

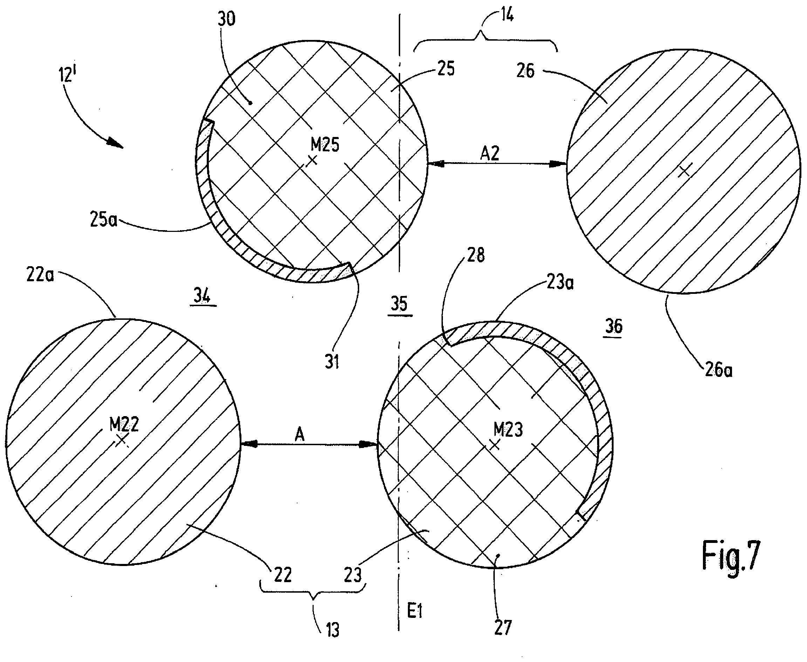

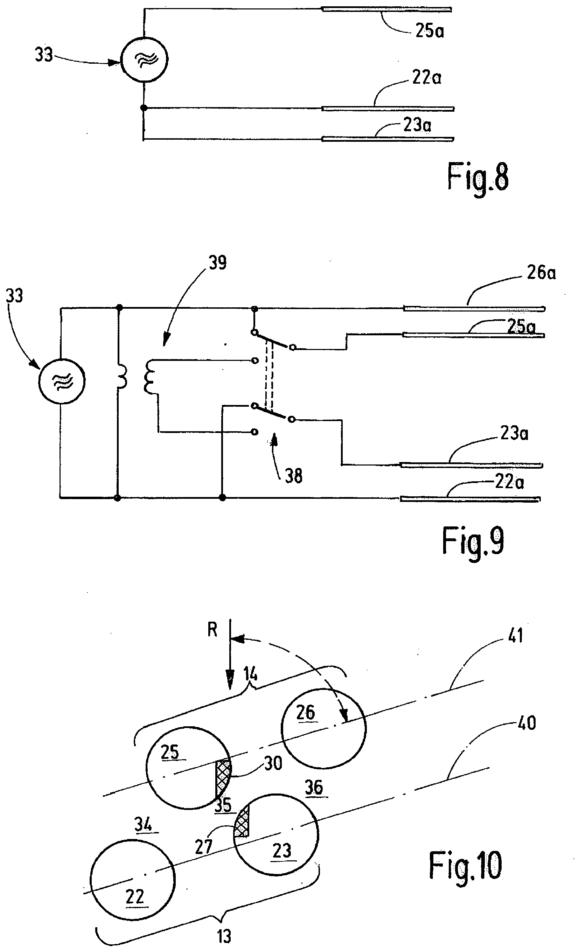

Die der Schenkelanordnung 13 zugeordnete Gegenschenkelanordnung 14 umfasst mindestens einen in Längsrichtung geraden oder leicht gekrümmten Gegenschenkel 25 und bei abgewandelten Ausführungsformen mehrere Gegenschenkel 25, 26, wie aus Figur 7 oder 10 hervorgeht. Der Gegenschenkel 25 weist eine elektrisch leitende Gegenelektrodenfläche 25a auf, deren Oberfläche im Querschnitt bogenförmig gekrümmt ist. Der Querschnitt des Gegenschenkels 25 kann entlang der Längsrichtung unveränderlich sei; alternativ kann sich der Gegenschenkel 25 in Längsrichtung auch leicht verjüngen.

An seiner dem Schenkel 23 benachbarten und von dem Schenkel 22 abliegenden Seite ist der Gegenschenkel 25 mit einem Isolator 30 versehen, der sich an eine Randkante 31 der Gegenelektrodenfläche 25a anschließt und in die Kontur des Gegenschenkels 25 einfügt. Der Isolator 30 überlappt, bezogen auf die Bewegungsrichtung R, den Isolator 27. Hingegen überlappt die von der Elektrodenfläche 23a und dem elektrisch leitenden Teil des Schenkels 23 gebildete Elektrode nicht mit der Elektrode, die von der Gegenelektrodenfläche 25a und dem elektrisch leitenden Teil des Gegenschenkels 25 gebildet ist.

Die in Figur 4 veranschaulichte vergrößerte Darstellung der Querschnitte der Schenkel 22, 23 und des Gegenschenkels 25 zeigt einige Maßbeziehungen derselben am Beispiel von Rundquerschnitten auf. Die Schenkel 22 und 23 weisen vorzugsweise gleiche oder auch leicht unterschiedliche Durchmesser D1 und D2 auf und sind in einem Abstand A zueinander angeordnet. Der Abstand A legt die lichte Weite zwischen den beiden Elektroden 22, 23 fest und wird quer zu einer Bewegungsrichtung R gemessen, in der der Gegenschenkel 25 relativ zu den Schenkeln 22, 23 und/oder die Schenkel 22, 23 relativ zu dem Gegenschenkel 25 beweglich sind.

Die Schenkel 22, 23 sind symmetrisch zu einer Mittellinie E angeordnet. Die Mittellinie E ist parallel zu der Bewegungsrichtung R orientiert und geht durch den Mittelpunkt M25 des Querschnitts des Gegenschenkels 25. Der Mittelpunkt M25 wird bei nichtkreisförmigen Querschnitten durch den Flächenschwerpunkt der Querschnittsfläche gebildet. Gleiches gilt für Mittelpunkte M22 und M23 der Querschnittsflächen der Schenkel 22, 23. Die Mittelpunkte M22, M23 liegen auf einer Linie L, die die Mittellinie vorzugsweise rechtwinklig schneidet. Die Linie L kann aber auch in einem spitzen Winkel zu der Mittellinie festgelegt sein, so dass der Abstand des Schenkels 23 von dem Gegenschenkel 25 geringer ist, als der Abstand des Schenkels 22 von dem Gegenschenkel 25. Diese Verhältnisse geben den geometrischen Idealfall wieder. Auf Grund von Produktionstoleranzen, Elastizitäten der Elemente und durch den Einsatz bedingten Verformungen kann es zu Abweichungen kommen.

Der Gegenschenkel 25 weist einen Durchmesser D3 auf, der vorzugsweise größer ist als der Abstand A und mittig symmetrisch auf der Mittelebene oder Mittellinie E angeordnet. Damit berührt die Gegenelektrode 25 mindestens eine der Elektroden 22, 23, vorzugsweise beide, wenn sie weitest möglich auf diese hin bewegt wird. Infolgedessen können die Schenkelanordnung 13 und die Gegenschenkelanordnung 14 sowohl große voluminöse als auch sehr filigrane feine Gefäße 32 zwischen einander fassen, wobei sich jeweils Verhältnisse entsprechend Figur 3 einstellen. Dies gilt insbesondere, wenn die Schenkel 22, 23 und/oder der Gegenschenkel 25 etwas federnd nachgiebig ausgebildet sind. Die Schenkel 22, 23 und der Gegenschenkel 25 können durch federnde Anpassung aneinander auch sehr kleine Gefäße koagulieren.

Die Elektrodenflächen 22a des Schenkels 22 ist eine mit dem Radius D1/2 gerundete Koagulationsfläche, die der Gegenelektrode 25 zugewandt ist. Die Gegenelektrodenfläche 25a der Gegenelektrode 25 ist mit dem Radius D3 gerundet und der Elektrodenfläche 22a zugewandt. Das Gefäß 32 ist zwischen der Elektrodenfläche 22a und der Gegenelektrodenfläche 25a gefasst und bestromt. Infolge der lediglich sanften Rundung der Elektrodenflächen 22a, 25a und der relativ großflächigen Berührung zwischen den Elektrodenflächen 22a, 25a und dem Gefäß 32 wird das Gefäß zwischen den Elektrodenflächen 22a, 25a bei geschlossenen Branchen komprimiert, wie es in Figur 3 veranschaulicht ist, und koaguliert.

Hingegen wird das Gefäß 32 zwischen dem Schenkel 23 und dem Gegenschenkel 25 zumindest teilweise zwischen den Isolatoren 27, 30 gefasst und komprimiert. Die Randkanten 28, 31 haben einen sehr geringen Krümmungsradius, der jeweils deutlich geringer ist, als die Krümmungsradien der Elektrodenflächen 22a, 23a, 25a. Der in Richtung der Linie L gemessene Abstand A1 zwischen den Randkanten 28, 31 beträgt vorzugsweise 0,1 mm bis 0,75 mm. Damit ist einerseits ein elektrischer Kurzschluss zwischen den Elektrodenflächen 23a, 25a ausgeschlossen und andererseits eine hohe Stromdichte in dem Gewebe erreichbar.

Zur Präparation von Organen, Tumoren, zum Schneiden von Gewebe oder auch zum (einseitigen) Schließen und Durchtrennen von Gefäßen kann mit dem insoweit beschriebenen Instrument 10 wie folgt vorgegangen werden:

Das Instrument 10 ist, wie es Figur 3 und 8 veranschaulichen, an eine elektrische Quelle 33 angeschlossen. Die Elektrodenflächen 22a, 23a sind mit einem Pol der Quelle 33 und die Gegenelektrodenfläche 25a mit einem anderen Pol derselben verbunden. Die elektrische Quelle 33 ist vorzugsweise ein HF-Generator, der eine Spannung mit einer Frequenz von mehreren 100 kHz und einer Spannung zwischen 80 Vₚ und 500 Vₚ abgibt (vorzugsweise zwischen 200 Vₚ, 250 Vₚ). In Benutzung wird das Gewebe 32 zwischen der Gegenelektrodenfläche 25a und den Elektrodenflächen 22a, 23a gefasst, wonach die Gegenschenkelanordnung 14 und die Schenkelanordnung 13 beim Schließen des Werkzeugs 12 so aufeinander zu bewegt werden, dass alle Lumen des Gewebes 32 geschlossen sind. Spätestens zu diesem Zeitpunkt oder kurz danach wird der Generator 33 aktiviert, so dass die beiden von der Gegenelektrodenfläche 25a und der Randkante 31 ausgehenden, durch das Gewebe 32 zu den Elektrodenflächen 22a, 23a führenden Strompfade bestromt werden und das dort komprimierte Gewebe durch Stromeinwirkung erhitzt, koaguliert und geschnitten wird. Die Elektrodenfläche 22a und Gegenelektrodenfläche 25a begrenzen somit einen Koagulationsspalt 34.

Dabei zeigt sich, dass mit der Elektrodenfläche 22a und der Gegenelektrodenfläche 25a Durchmessern D1, D3 von 0,3 mm bis 1,5 mm vorzugsweise 0,4 mm bis 1 mm eine Gefäßversiegelung mit hohem Berstdruck (von z.B. über 120 mm Hg) in kurzer Zeit (z.B. unter 4 s) erreicht werden kann.

Die Elektrodenflächen 23a, 25a sind lediglich jeweils einseitig mit der Randkante 28 bzw. 31 versehen, was zu einer Stromkonzentration und somit zur Ausbildung eines Schneidspalts 35 zwischen den Isolatoren 27, 30 führt. Die gleiche Spannung führt bei gleicher Behandlungsdauer und gleichzeitigem Aktivieren in dem Koagulationsspalt zur Gewebefusion und in dem Schneidspalt 35 zur Gewebedissektion. Alternativ kann auch in einer ersten Phase im Wesentlichen nur koaguliert und in einer zweiten Phase im Wesentlichen nur geschnitten werden.

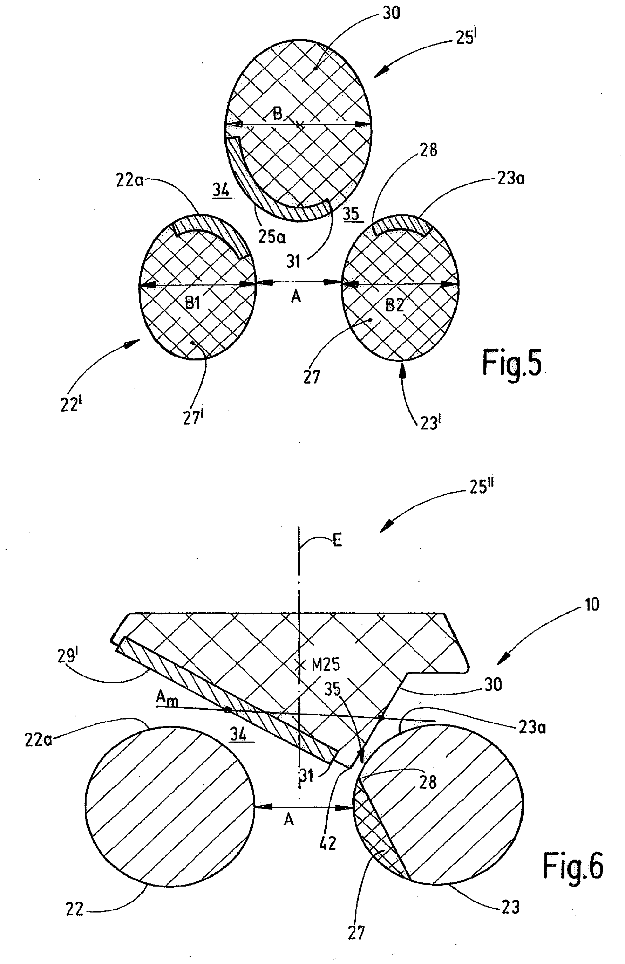

Die Schenkel 22, 23 und/oder der Gegenschenkel 25 können in ihrem jeweiligen Querschnitt von der Kreisform abweichen, wie beispielsweise aus Figur 5 erkennbar ist. Außerdem ist es möglich, die Schenkel 22 und 23' und/oder den Gegenschenkel 25' jeweils als Isolator 27, 27', 30 auszubilden und die Elektrodenflächen 22a, 23a und/oder die Gegenelektrodenfläche 25a an elektrisch leitenden Beschichtungen oder Metallinlays auszubilden, die von den isolierend ausgebildeten Schenkeln 22', 23' und/oder dem aus isolierendem Material ausgebildeten Gegenschenkel 25' getragen werden. Bei dem abgewandelten Instrument 10 nach Figur 5 ist der Abstand A wiederum geringer als die in gleicher Richtung gemessene Breite B der Gegenelektrode 25'. Vorzugsweise sind auch die Breiten B1, B2 der Schenkel 22', 23', die in gleicher Richtung wie der Abstand A1 und die Breite B zu messen sind, größer als der Abstand A. Ansonsten gilt die zuvor zu den Figuren 1 bis 4 und 8 gegebene Beschreibung entsprechend.

Ein weiter abgewandeltes Ausführungsbeispiel zeigt Figur 6. Die Schenkel 22, 23 können dort jede der vorgenannten Formen zum Beispiel nach Figur 4 oder nach Figur 5 aufweisen. Die Gegenelektrode 25" weist hier zwei nichtkonkave, im Beispiel ebene Abschnitte 25a', 30' auf, wobei die Verbindung 42 zwischen ihnen durch eine Ecke bzw. Kante mit geringem Krümmungsradius gebildet sein kann. Die Abschnitte 25a', 30' weisen einen Mittenabstand Am auf, der wiederum größer ist als der Abstand A zwischen den Schenkeln 22, 23. Auch mit dieser Konfiguration lassen sich die oben skizzierten vorteilhaften Wirkungen des erfindungsgemäßen Konzepts erzielen.

Allen Ausführungsformen des Instruments 10 ist gemeinsam, dass die Gegenelektrodenfläche 25a zu der Mittellinie E außermittig angeordnet ist. Figur 6 veranschaulicht dies an einem Instrument 10 mit flacher Gegenelektrodenfläche 25a. Der Isolator 27 kann wie dargestellt vorhanden sein oder alternativ auch entfallen. Er fördert aber die Schneidwirkung.

Eine weiter entwickelte Ausführungsform zeigt Figur 7. Soweit diese mit der Ausführungsform nach Figur 2 bis 5 übereinstimmt, wird auf die diesbezüglichen Beschreibungsteile unter Zugrundelegung gleicher Bezugszeichen verwiesen. Ergänzend gilt:

Während die Schenkel 22, 23 in Figur 4 symmetrisch zu der Mittelebene E oder Mittellinie E angeordnet sind, verläuft die Mittelebene oder Mittellinie E1 bei der Ausführungsform nach Figur 7 vorzugsweise außermittig durch den Gegenschenkel 25 und den Schenkel 23. Die Schenkelanordnung 13 und die Gegenschenkelanordnung 14 bilden gemäß Figur 7 eine asymmetrische Anordnung. Die Gegenschenkelanordnung 14 umfasst ein Paar Gegenschenkel 25, 26, das gegen das Paar Schenkel 22, 23 seitlich versetzt ist, wobei sich beim Schließen der Gegenschenkel 25 zwischen den Schenkeln 22, 23 und der Schenkel 23 zwischen den Gegenschenkeln 25, 26 zentriert. Der Abstand A2 zwischen den Gegenschenkeln 25, 26 kann ebenso groß wie der Abstand A zwischen den Schenkeln 22, 23 oder auch, abweichend von diesem, insbesondere etwas größer gewählt sein. Hinsichtlich der Form und Anordnung der Schenkel 22, 23 sowie der Form und Anordnung der Gegenschenkel 25, 26 gelten alle obigen Ausführungen und Möglichkeiten entsprechend.

Mit der Konfiguration nach Figur 7 werden insgesamt zwei Koagulationszonen 34, 36 und dazwischen ein Schneidspalt definiert, nämlich jeweils paarweise zwischen dem Schenkel 22 und dem Gegenschenkel 25 (Koagulationsbereich 34); zwischen dem Gegenschenkel 25 und dem Schenkel 23 (Schneidbereich 35); zwischen dem Schenkel 23 und dem Gegenschenkel 26 (Koagulationsbereich 36). Mit einer solchen Elektrodenkonfiguration lassen sich Gefäße fusionieren und Gewebe koagulieren. Dabei können die Elektrodenflächen 22a, 23a an einem Pol und die Gegenelektrodenflächen 25a, 26a an einem anderen Pol der Quelle 33 angeschlossen sein.

Bei einer weiter abgewandelten Ausführungsform ist es jedoch möglich, die Koagulationsbereiche 34, 36 und den Schneidbereich 35 unterschiedlich zu bestromen. Dazu kann zum Beispiel eine Versorgungsschaltung nach Figur 9 dienen. Diese enthält eine Umschalteinrichtung 38, mit der die Elektrodenfläche 23a und die Gegenelektrodenfläche 25a bedarfsweise entweder an die gleiche Versorgungsspannung angelegt werden, wie die Elektrodenflächen 22a und die Gegenelektrodenfläche 26a, oder an eine höhere Versorgungsspannung. Diese kann durch eine Quelle 39 bereitgestellt werden, die beispielsweise durch einen Transformator gebildet wird. Dieser kann in dem Gehäuse 18 oder auch in dem Gerät 20 angeordnet sein.

Zum Beispiel läuft der Betrieb eines Werkzeugs 12' nach Figur 7 zum Beispiel wie folgt ab:

Zunächst wird ein biologisches Gewebe, z.B. ein Gefäß zwischen der Schenkelanordnung 13 und der Gegenschenkelanordnung 14 gefasst und komprimiert. Ist dies geschehen, werden die Elektrodenflächen 22a, 23a mit einem Pol der Quelle 33 und die Gegenelektrodenflächen 25a, 26a mit einem anderen Pol der Quelle 33 verbunden. In den Zonen 34, 36 findet nun eine Gefäßkoagulation statt. Durch die Stromkonzentration an den Randkanten 28, 31 findet in der Schneidzone 35 eine auf einen kleinen Bereich konzentrierte Koagulation, Austrocknung und Durchtrennung des Gewebes statt.

Zur Unterstützung des Schneidvorgangs können nach Durchführung der Koagulation die Elektrodenfläche 23a und die Gegenelektrodenfläche 25a mit einer höheren Spannung beaufschlagt werden, indem die Umschalteinrichtung 38 aktiviert wird. Zugleich können die Elektrodenfläche 22a und die Gegenelektrodenfläche 26a deaktiviert oder (auch weiter) betrieben werden. Durch die höhere Spannung in dem Schneidbereich 35 kann dort nun bereits koaguliertes Gewebe durchtrennt werden, wobei der sich bildende Schnitt von den Koagulationssäumen eingefasst ist, die in den Koagulationszonen 34 und 36 gebildet worden sind.

Auf diese Weise wird ein schneidelektrodenloses und ein messerloses Fusionsinstrument geschaffen, das selbstzentrierend ausgebildet und äußerst filigran gestaltet werden kann.

Figur 10 veranschaulicht eine weitere Abwandlung eines Fusionsinstruments mit zwei im Querschnitt z.B. kreisförmigen Schenkeln 22, 23 und zwei z.B. ebenfalls kreisförmigen Gegenschenkeln 25, 26. Die Schenkel 22, 23 sind auf einer Linie 40 angeordnet, die in einem spitzen Winkel zu der Bewegungsrichtung B orientiert ist. Gleichfalls sind die Gegenschenkel 25, 26 auf einer Linie 41 angeordnet, die ebenfalls schräg zu der Bewegungsrichtung B und vorzugsweise parallel zu der Linie 40 angeordnet ist. Bei dieser Konfiguration ist der Schneidspalt enger als die Koagulationsspalte 34, 36. Dadurch und durch die Wirkung der Isolatoren 27, 30 kann die Stromdichte in dem mittleren Schneidspalt 35 größer sein, als in den seitlichen Koagulationsspalten 34, 36, so dass in dem Schneidspalt 35 eine schnelle und sichere Durchtrennung des Gewebes provoziert werden kann.

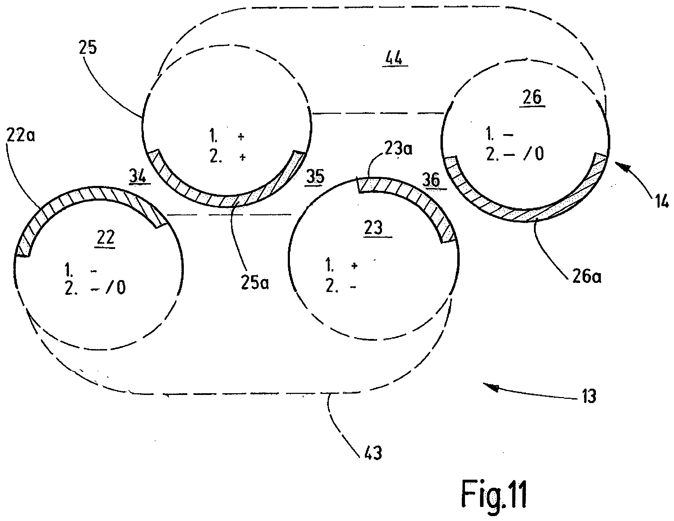

Figur 11 veranschaulicht eine bauliche Abwandlungen, die bei allen vorstehend beschriebenen Instrumenten zur Anwendung kommen können. Es ist nämlich möglich, die Schenkel 22, 23 baulich zu vereinen, indem zwischen beiden eine Brücke 43 ausgebildet wird. Z.B. werden die Schenkel 22, 23 und die Brücke 43 durch ein Kunststoffformteil gebildet, das mit Blechen oder elektrisch leitenden Formteilen versehen ist, die Elektrodenflächen 22a, 23a bilden. Zusätzlich oder alternativ können die Gegenschenkel 25, 26 durch eine Brücke 44 verbunden sein, die mit den Gegenschenkeln 25, 26 ein mit Elektroden 25a, 26a versehenes Formteil z.B. aus Kunststoff bildet. Die Elektroden 25a, 26a können wiederum Bleche oder elektrisch leitende Formteile sein. An der Schenkelanordnung 13 werden die Schenkel 22, 23 durch Bereiche oder leicht erhabene rippenartige Abschnitte des Formteils gebildet. An der Gegenschenkelanordnung 14 werden die Schenkel 25, 26 ebenfalls durch Bereiche oder leicht erhabene rippenartige Abschnitte des Formteils gebildet.

Aus Figur 11 gehen zudem vorteilhafte Abläufe eines Koagulations- und Schneidvorgangs hervor, der auch bei den Instrumenten nach 7 und 10 Anwendung finden kann:

Zunächst wird eine Koagulation mit einer HF-Beaufschlagung des Instruments gemäß der bei 1. angegebenen Polung vorgenommen. Damit werden die Koagulationsspalte 34 und 36 elektrisch durchströmt, während der Schneidspalt 35 stromlos bleibt. Ein erster Pol der Quelle liegt an der Elektrode 23a und der Elektrode 25a des Gegenschenkels 25, während der andere Pol der Quelle an der Elektrode 22a und der Gegenelektrode 26a des Gegenschenkels 26 liegt. Während das biologische Gewebe in den Koagulationsspalten 34, 36 koaguliert und fusioniert wird, bleibt es in dem Schneidspalt 35 zunächst frisch. ####

Schnitt mit einem Pol auf 25a und dem anderen Pol auf 23a. Die Elektroden 22a und 26a floaten während des Schnittes, welcher in diesem Fall nachgelagert erfolgen kann.

Der Unterschied mag nicht wichtig erscheinen, es ist jedoch so, dass zuvor koaguliertes Gewebe nur mehr schlecht elektrisch geschnitten werden kann. Es ist daher mit entscheidend, das Gewebe im Bereich 35 vor dem Schnitt möglichst wenig auszutrocknen.

Das erfindungsgemäße Instrument 10 weist eine Schenkelanordnung 13 mit zwei Schenkeln 22, 23 auf, die an ihren der Gegenschenkelanordnung 14 zugewandten Seite gerundete und/oder auch flache Elektrodenflächen 22a, 23a aufweisen. Die Gegenschenkelanordnung 14 umfasst wenigstens eine Gegenelektrodenfläche 25a, die ebenfalls gerundet und/oder flach ausgebildet ist. Eine der Elektrodenflächen 22a, 23a weist eine vorzugsweise einseitig von einem Isolator 27 eingefasste Randkante 28 auf, die der benachbarten Elektrodenfläche 22a der Schenkelanordnung 13 benachbart ist. Die Gegenelektrodenfläche 25a weist gleichfalls eine einem Isolator 30 benachbarte Randkante 31 auf, die der Randkante 28 zugewandt ist. Die Randkanten 28, 31 der als Koagulationsflächen dienenden Elektrodenflächen 23a, 25a sind einander so nahe (0,1 mm bis 0,75 mm), dass sie Schneidkanten bilden, die im Idealfalle schon mit sonst nur zur Koagulation tauglichen niedrigen Spannungen eine Schneidwirkung entfalten. Mit diesem Konzept lassen sich im Längsverlauf gekrümmte, äußerst filigrane Fusionsinstrumente mit geringer thermischen Trägheit und sehr guter Fusions- und Schneidwirkung bereitstellen.

**Bezugszeichen:**

| | |
|---|---|
| 10 | Instrument |
| 11 | Schaft |
| 12, 12' | Werkzeug |
| 14 | Gegenschenkelanordnung |
| 13 | Schenkelanordnung |
| 15 | Achse |
| 16 | Griff |
| 17 | Betätigungshebel |
| 18 | Betätigungshebel |
| 19 | Kabel |
| 20 | Gerät |
| 22, 22' | erster Schenkel |
| 22a | Elektrodenfläche des ersten Schenkels 22 |
| 23, 23' | zweiter Schenkel |
| 23a | Elektrodenfläche des zweiten Schenkels 23 |
| 25, 25', 25" | erster Gegenschenkel |
| 25a, 25a' | Gegenelektrodenfläche des Gegenschenkels 25, 25' |
| 26 | zweiter Gegenschenkel |
| D1 | Durchmesser des Querschnitts des ersten Schenkels 22 |
| D2 | Durchmesser des Querschnitts des zweiten Schenkels 23 |
| A | Abstand der Schenkel 22, 23 voneinander |
| A1 | Abstand der Randkanten 28, 30 voneinander |
| A2 | Abstand der Gegenschenkel 25, 26 voneinander |
| R | Bewegungsrichtung |
| E, E1 | Mittellinie |
| D3 | Durchmesser des Querschnitts des Gegenschenkels 25 |
| 27 | Isolator |
| 28 | Randkante |
| 30 | Isolator |
| 31 | Randkante |
| 32 | Gewebe, Gefäß |
| 33 | Quelle |
| M | Mittenabstand |
| 34 | linker Koagulationsbereich |
| 35 | Schneidbereich |
| 36 | rechter Koagulationsbereich |
| 26a | Gegenelektrodenfläche des Gegenschenkels 26 |
| 38 | Umschalteinrichtung |
| 39 | Transformator |
| 40, 41 | Linie |
| 42 | Verbindung bzw. Ecke an polygonalem Gegenschenkel 25" |
| Am | Mittenabstand in Figur 6 |
| | |
| | |

## Patentansprüche

1. Instrument (10), insbesondere zur Gefäßfusion,
mit einer Schenkelanordnung (13), die einen ersten Schenkel (22) mit einer ersten Elektrodenfläche (22a) und einen zweiten Schenkel (23) mit einer zweiten Elektrodenfläche (23a) aufweist, die in einem Abstand zueinander gehalten sind,
mit einer Gegenschenkelanordnung (14), die wenigstens einen Gegenschenkel (25, 26) aufweist,
wobei der erste Schenkel (22), der zweite Schenkel (23) und/oder der Gegenschenkel (25) eine Elektrodenfläche (25a) aufweist, die zu einer Mittellinie (E) außermittig angeordnet ist, die durch Bewegungsrichtung (R) des Schenkels (22, 23) oder Gegenschenkels (25) und dessen Querschnittsmittelpunkt (M22, M23, M25) festgelegt ist.

2. Instrument nach Anspruch 1, **dadurch gekennzeichnet, dass** der zweite Schenkel (23) der Schenkelanordnung (13) eine elektrisch leitende Elektrodenfläche (23a) und einen isolierten Abschnitt (27) aufweist, der auf der dem ersten Schenkel (22) zugewandten Seite des zweiten Schenkels (23) angeordnet ist.

3. Instrument nach Anspruch 1 oder 2, **dadurch gekennzeichnet, dass** der Gegenschenkel (25) eine elektrisch leitende Gegenelektrodenfläche (25a) und einen elektrisch isolierten Gegenabschnitt (30) aufweist, der auf der dem ersten Schenkel (22) abgewandten Seite angeordnet ist.

4. Instrument nach einem der vorstehenden Ansprüche, **dadurch gekennzeichnet, dass** der Gegenschenkel (25) eine konvex gewölbte Oberfläche aufweist, die den Schenkeln (22, 23) zugewandt ist, und dass die Schenkel (22, 23) jeweils eine dem Gegenschenkel (25) zugewandte konvex gekrümmte Oberfläche aufweisen, wobei vorzugsweise der Abstand (A) zwischen dem ersten Schenkel (22) und dem zweiten Schenkel (23) voneinander geringer ist, als der Durchmesser des Gegenschenkels (25).

5. Instrument nach einem der vorstehenden Ansprüche, **dadurch gekennzeichnet, dass** der Gegenschenkel (25) einen Mittelpunkt (M25) aufweist, der auf einer Mittellinie (E) angeordnet ist, zu denen auch die Schenkel (22, 23) der Schenkelanordnung auf verschiedenen Seiten in gleichen Abständen angeordnet sind.

6. Instrument nach einem der vorstehenden Ansprüche, **dadurch gekennzeichnet, dass** die Querschnittsfläche des Gegenschenkels (25) höchstens so groß ist, wie die Summe der beiden Querschnittsflächen der beiden Schenkel (22, 23).

7. Instrument nach einem der vorstehenden Ansprüche 2 bis 6, **dadurch gekennzeichnet, dass** die elektrisch leitende Elektrodenfläche (23a) des zweiten Schenkels (23) der Schenkelanordnung (13) in Nachbarschaft zu dem isolierten Abschnitt (27) eine stromkonzentrierende Randkante (28) aufweist.

8. Instrument nach einem der vorstehenden Ansprüche 2 bis 7, **dadurch gekennzeichnet, dass** die elektrisch leitende Elektrodenfläche (25a) des ersten Gegenschenkels (25) der Gegenschenkelanordnung (14) in Nachbarschaft zu dem isolierten Abschnitt (30) eine stromkonzentrierende Randkante (31) aufweist.

9. Instrument nach einem der vorstehenden Ansprüche 2 bis 8, **dadurch gekennzeichnet, dass** die erste Elektrodenfläche (22a) und die zweite Elektrodenfläche (23a) elektrisch untereinander verbunden sind.

10. Instrument nach einem der vorstehenden Ansprüche, **dadurch gekennzeichnet, dass** zu der Gegenschenkelanordnung (14) zwei Gegenschenkel (25, 26) gehören, die in einem Abstand zueinander gehalten sind.

11. Instrument nach einem der vorstehenden Ansprüche, **dadurch gekennzeichnet, dass** die Schenkel (22, 23) der Schenkelanordnung (13) und die Gegenschenkel (25, 26) der Gegenschenkelanordnung (14) seitlich gegeneinander versetzt angeordnet sind.

12. Instrument nach einem der Ansprüche 10 oder 11, **dadurch gekennzeichnet, dass** die Gegenschenkel (25, 26) miteinander elektrisch verbundene Gegenelektrodenflächen (25a, 26a) aufweisen.

13. Instrument nach einem der vorstehenden Ansprüche, **dadurch gekennzeichnet, dass** die Elektrodenflächen (22a, 23a) der Schenkelanordnung (13) mit unterschiedlichen Spannungen beaufschlagbar sind.

14. Instrument nach einem der vorstehenden Ansprüche, **dadurch gekennzeichnet, dass** die Gegenschenkelanordnung (14) zwei Gegenelektroden (25, 26) umfasst, die mit unterschiedlichen Spannungen beaufschlagbar sind.

15. Instrument nach einem der vorstehenden Ansprüche, **dadurch gekennzeichnet, dass** die Gegenschenkelanordnung (14) zwei Gegenelektrodenflächen (25a, 26a) umfasst, die mit den Elektrodenflächen (22a, 23a) zwei Koagulationsspalte (34, 36) und einen Schneidspalt (35) festlegen.
